# EUROPEAN PATENT APPLICATION

(11) **EP 2 712 916 A2**
(43) Date of publication of application: **02.04.2014**
(21) Application number: 13196997.4
(22) Date of filing: 06.06.2008
(51) Int. Cl.: C11D 3/386, D06M 16/00, C12N 9/08, C12N 9/42, C11D 3/00, C11D 3/04

(54) **An enzyme composition for combined biopolishing and bleach clean-up**

(30) Priority: 11.06.2007 EP 07109969
(62) Divisional of application: 08760676.0
(71) Applicant: Novozymes A/S, 2880 Bagsvaerd (DK)
(72) Inventor: Kuilderd, Harm, Albertus, 100103 Beijing (CN); Wu, Guifang, 100085 Beijing (CN); Li, Haijing, 100085 Beijing (CN); Zhou, Quan, 100085 Beijing (CN)
(74) Representative: Thomas, Philip John Duval

(57) **Abstract**

The present application provides compositions for treating textile, wherein the textile is treated by a system for removing hydrogen peroxide and an enzyme system for bio-polishing in one step to achieve the biopolishing and bleach clean up effect.

SEQUENCE LISTING

<110>  Novozymes A/S

<120>  A Process for Combined BioPolishing and Bleach Clean-up

<130>  NOVBT/P54740EPdiv1

<160>  3

<170>  PatentIn version 3.4

<210>  1
<211>  299
<212>  PRT
<213>  Thielavia terrestis

<400>  1

Met Arg Ser Thr Pro Val Leu Arg Thr Thr Leu Ala Ala Ala Leu Pro
1               5                   10                  15

Leu Val Ala Ser Ala Ala Ser Gly Ser Gly Gln Ser Thr Arg Tyr Trp
            20                  25                  30

Asp Cys Cys Lys Pro Ser Cys Ala Trp Pro Gly Lys Ala Ala Val Ser
        35                  40                  45

Gln Pro Val Tyr Ala Cys Asp Ala Asn Phe Gln Arg Leu Ser Asp Phe
    50                  55                  60

Asn Val Gln Ser Gly Cys Asn Gly Gly Ser Ala Tyr Ser Cys Ala Asp
65                  70                  75                  80

Gln Thr Pro Trp Ala Val Asn Asp Asn Leu Ala Tyr Gly Phe Ala Ala
                85                  90                  95

Thr Ser Ile Ala Gly Gly Ser Glu Ser Ser Trp Cys Cys Ala Cys Tyr
            100                 105                 110

Ala Leu Thr Phe Thr Ser Gly Pro Val Ala Gly Lys Thr Met Val Val
        115                 120                 125

Gln Ser Thr Ser Thr Gly Gly Asp Leu Gly Ser Asn Gln Phe Asp Ile
    130                 135                 140

Ala Met Pro Gly Gly Gly Val Gly Ile Phe Asn Gly Cys Ser Ser Gln
145                 150                 155                 160

Phe Gly Gly Leu Pro Gly Ala Gln Tyr Gly Gly Ile Ser Ser Arg Asp
                165                 170                 175

Gln Cys Asp Ser Phe Pro Ala Pro Leu Lys Pro Gly Cys Gln Trp Arg
            180                 185                 190

Phe Asp Trp Phe Gln Asn Ala Asp Asn Pro Thr Phe Thr Phe Gln Gln
        195                 200                 205

Val Gln Cys Pro Ala Glu Ile Val Ala Arg Ser Gly Cys Lys Arg Asn
    210                 215                 220

Asp Asp Ser Ser Phe Pro Val Phe Thr Pro Pro Ser Gly Gly Asn Gly
225                 230                 235                 240

Gly Thr Gly Thr Pro Thr Ser Thr Ala Pro Gly Ser Gly Gln Thr Ser
                245                 250                 255

Pro Gly Gly Gly Ser Gly Cys Thr Ser Gln Lys Trp Ala Gln Cys Gly
            260                 265                 270

Gly Ile Gly Phe Ser Gly Cys Thr Thr Cys Val Ser Gly Thr Thr Cys
        275                 280                 285

Gln Lys Leu Asn Asp Tyr Tyr Ser Gln Cys Leu
    290                 295

<210> 2
<211> 305
<212> PRT
<213> Humicola insolens

<400> 2

Met Arg Ser Ser Pro Leu Leu Arg Ser Ala Val Val Ala Ala Leu Pro
1               5                   10                  15

Val Leu Ala Leu Ala Ala Asp Gly Arg Ser Thr Arg Tyr Trp Asp Cys
            20                  25                  30

Cys Lys Pro Ser Cys Gly Trp Ala Lys Lys Ala Pro Val Asn Gln Pro
        35                  40                  45

Val Phe Ser Cys Asn Ala Asn Phe Gln Arg Ile Thr Asp Phe Asp Ala
    50                  55                  60

Lys Ser Gly Cys Glu Pro Gly Gly Val Ala Tyr Ser Cys Ala Asp Gln
65                  70                  75                  80

Thr Pro Trp Ala Val Asn Asp Asp Phe Ala Leu Gly Phe Ala Ala Thr
                85                  90                  95

Ser Ile Ala Gly Ser Asn Glu Ala Gly Trp Cys Cys Ala Cys Tyr Glu
        100                 105                 110

Leu Thr Phe Thr Ser Gly Pro Val Ala Gly Lys Lys Met Val Val Gln
    115                 120                 125

Ser Thr Ser Thr Gly Gly Asp Leu Gly Ser Asn His Phe Asp Leu Asn
    130                 135                 140

Ile Pro Gly Gly Gly Val Gly Ile Phe Asp Gly Cys Thr Pro Gln Phe
145                 150                 155                 160

Gly Gly Leu Pro Gly Gln Arg Tyr Gly Gly Ile Ser Ser Arg Asn Glu
                165                 170                 175

Cys Asp Arg Phe Pro Asp Ala Leu Lys Pro Gly Cys Tyr Trp Arg Phe
        180                 185                 190

Asp Trp Phe Lys Asn Ala Asp Asn Pro Ser Phe Ser Phe Arg Gln Val
    195                 200                 205

Gln Cys Pro Ala Glu Leu Val Ala Arg Thr Gly Cys Arg Arg Asn Asp
    210                 215                 220

Asp Gly Asn Phe Pro Ala Val Gln Ile Pro Ser Ser Ser Thr Ser Ser
225                 230                 235                 240

Pro Val Asn Gln Pro Thr Ser Thr Ser Thr Thr Ser Thr Ser Thr Thr
                245                 250                 255

Ser Ser Pro Pro Val Gln Pro Thr Thr Pro Ser Gly Cys Thr Ala Glu
        260                 265                 270

Arg Trp Ala Gln Cys Gly Gly Asn Gly Trp Ser Gly Cys Thr Thr Cys
    275                 280                 285

Val Ala Gly Ser Thr Cys Thr Lys Ile Asn Asp Trp Tyr His Gln Cys
    290                 295                 300

Leu
305

<210>  3
<211>  717
<212>  PRT
<213>  Scytalidium thermophilum

<400>  3

Met Asn Arg Val Thr Asn Leu Leu Ala Trp Ala Gly Ala Ile Gly Leu
1               5                   10                  15

Ala Gln Ala Thr Cys Pro Phe Ala Asp Pro Ala Ala Leu Tyr Ser Arg
            20                  25                  30

Gln Asp Thr Thr Ser Gly Gln Ser Pro Leu Ala Ala Tyr Glu Val Asp
        35                  40                  45

Asp Ser Thr Gly Tyr Leu Thr Ser Asp Val Gly Gly Pro Ile Gln Asp
    50                  55                  60

Gln Thr Ser Leu Lys Ala Gly Ile Arg Gly Pro Thr Leu Leu Glu Asp
65                  70                  75                  80

Phe Met Phe Arg Gln Lys Ile Gln His Phe Asp His Glu Arg Val Pro
                85                  90                  95

Glu Arg Ala Val His Ala Arg Gly Ala Gly Ala His Gly Thr Phe Thr
            100                 105                 110

Ser Tyr Ala Asp Trp Ser Asn Ile Thr Ala Ala Ser Phe Leu Asn Ala
        115                 120                 125

Thr Gly Lys Gln Thr Pro Val Phe Val Arg Phe Ser Thr Val Ala Gly
    130                 135                 140

Ser Arg Gly Ser Ala Asp Thr Ala Arg Asp Val His Gly Phe Ala Thr
145                 150                 155                 160

Arg Phe Tyr Thr Asp Glu Gly Asn Phe Asp Ile Val Gly Asn Asn Ile
                165                 170                 175

Pro Val Phe Phe Ile Gln Asp Ala Ile Gln Phe Pro Asp Leu Ile His
            180                 185                 190

Ser Val Lys Pro Arg Pro Asp Asn Glu Ile Pro Gln Ala Ala Thr Ala
        195                 200                 205

His Asp Ser Ala Trp Asp Phe Phe Ser Gln Gln Pro Ser Thr Met His
    210                 215                 220

Thr Leu Phe Trp Ala Met Ser Gly His Gly Ile Pro Arg Ser Tyr Arg
225                 230                 235                 240

His Met Asp Gly Phe Gly Val His Thr Phe Arg Phe Val Lys Asp Asp
                245                 250                 255

Gly Ser Ser Lys Leu Ile Lys Trp His Phe Lys Ser Arg Gln Gly Lys
            260                 265                 270

Ala Ser Leu Val Trp Glu Glu Ala Gln Val Leu Ser Gly Lys Asn Ala
        275                 280                 285

Asp Phe His Arg Gln Asp Leu Trp Asp Ala Ile Glu Ser Gly Asn Gly
    290                 295                 300

Pro Glu Trp Asp Val Cys Val Gln Ile Val Asp Glu Ser Gln Ala Gln
305                 310                 315                 320

Ala Phe Gly Phe Asp Leu Leu Asp Pro Thr Lys Ile Ile Pro Glu Glu
                325                 330                 335

Tyr Ala Pro Leu Thr Lys Leu Gly Leu Leu Lys Leu Asp Arg Asn Pro
            340                 345                 350

Thr Asn Tyr Phe Ala Glu Thr Glu Gln Val Met Phe Gln Pro Gly His
        355                 360                 365

Ile Val Arg Gly Ile Asp Phe Thr Glu Asp Pro Leu Leu Gln Gly Arg
    370                 375                 380

Leu Phe Ser Tyr Leu Asp Thr Gln Leu Asn Arg Asn Gly Gly Pro Asn
385                 390                 395                 400

Phe Glu Gln Leu Pro Ile Asn Met Pro Arg Val Pro Ile His Asn Asn
                405                 410                 415

Asn Arg Asp Gly Ala Gly Gln Met Phe Ile His Arg Asn Lys Tyr Pro
            420                 425                 430

Tyr Thr Pro Asn Thr Leu Asn Ser Gly Tyr Pro Arg Gln Ala Asn Gln
        435                 440                 445

Asn Ala Gly Arg Gly Phe Phe Thr Ala Pro Gly Arg Thr Ala Ser Gly
    450                 455                 460

Ala Leu Val Arg Glu Val Ser Pro Thr Phe Asn Asp His Trp Ser Gln
465                 470                 475                 480

Pro Arg Leu Phe Phe Asn Ser Leu Thr Pro Val Glu Gln Gln Phe Leu
                485                 490                 495

Val Asn Ala Met Arg Phe Glu Ile Ser Leu Val Lys Ser Glu Glu Val
            500                 505                 510

Lys Lys Asn Val Leu Thr Gln Leu Asn Arg Val Ser His Asp Val Ala
        515                 520                 525

Val Arg Val Ala Ala Ala Ile Gly Leu Gly Ala Pro Asp Ala Asp Asp
    530                 535                 540

Thr Tyr Tyr His Asn Asn Lys Thr Ala Gly Val Ser Ile Val Gly Ser
545                 550                 555                 560

Gly Pro Leu Pro Thr Ile Lys Thr Leu Arg Val Gly Ile Leu Ala Thr
                565                 570                 575

Thr Ser Glu Ser Ser Ala Leu Asp Gln Ala Ala Gln Leu Arg Thr Arg
            580                 585                 590

Leu Glu Lys Asp Gly Leu Val Val Thr Val Val Ala Glu Thr Leu Arg
        595                 600                 605

Glu Gly Val Asp Gln Thr Tyr Ser Thr Ala Asp Ala Thr Gly Phe Asp
    610                 615                 620

Gly Val Val Val Val Asp Gly Ala Ala Ala Leu Phe Ala Ser Thr Ala
625                 630                 635                 640

Ser Ser Pro Leu Phe Pro Thr Gly Arg Pro Leu Gln Ile Phe Val Asp
                645                 650                 655

Ala Tyr Arg Trp Gly Lys Pro Val Gly Val Cys Gly Gly Lys Ser Ser
            660                 665                 670

Glu Val Leu Asp Ala Ala Asp Val Pro Glu Asp Gly Asp Gly Val Tyr
        675                 680                 685

Ser Glu Glu Ser Val Asp Met Phe Val Glu Glu Phe Glu Lys Gly Leu
    690                 695                 700

Ala Thr Phe Arg Phe Thr Asp Arg Phe Ala Leu Asp Ser
705                 710                 715

## Description

### Reference to a Sequence Listing

This application contains a Sequence Listing in computer readable form. The computer readable form is incorporated herein by reference.

### Field of the Invention

The present invention relates to methods and compositions for treating textile, and more particularly, to methods and compositions for one-step combined biopolishing and bleach clean-up, comprising treating textile with a system for removing hydrogen peroxide and an enzyme system for bio-polishing.

### Background of the Invention

Usually, there is a process to remove the residual hydrogen peroxide after Bleaching. There are three kinds of way to do it. One is washing with water several times, one is treating the bath with reducing agents and the other is with the use of catalase. The using of catalase has become more and more popular. The preparation process, which may involve desizing (for woven goods), scouring, bleaching and bleach clean up, produces a textile suitable for dyeing. Bio-polishing is done before or after dyeing. Up to now, the Bleach Clean up, BioPolishing and dyeing are done in separate steps.

A. BioPolishing: Bio-Polishing is a specific treatment of the yarn surface which improves fabric quality with respect to handle and appearance. The most important effects of BioPolishing can be characterized by less fuzz and pilling, increased gloss/luster, improved fabric handle, increased durable softness and improved water absorbency. Bio-Polishing usually takes place in the wet processing of the manufacture of knitted and woven fabrics. Wet processing comprises such steps as e.g. desizing, scouring, bleaching, washing, dying/printing and finishing.

B. Bleach Clean-up: The purpose of bleaching is to completely remove colored impurities, improve absorbency, and achieve adequate whiteness and dyeability. In order to obtain clear, pure tones on a variety of fibres (particularly on natural fibres), it is necessary to bleach them prior to dyeing. This enables one to obtain a good ground fibre. From an ecological and technical point of view, hydrogen peroxide, its derivatives and addition products that release peroxide are generally used for the bleaching process. However, generally, excess peroxide product remains on the fibre and when this occurs it can interfere with and have an adverse affect on subsequent dyeings with anionic dyes, for example, reactive dyes where the dye is in part or totally destroyed. Destroying excess H₂O₂ after bleaching is referred to as the Bleach Clean-up process. This can be done by applying a catalase to the substrate. In order to obtain consistent, high quality results with commercial quantities of textiles, the Bleach Clean-up or bioPolishing steps are usually performed separately because it is very difficult to combine the enzymatic processes due to processing constraints related to pH.

C. Dyeing: Dyeing of textiles is often considered to be the most important and expensive single step in the manufacturing of textile fabrics and garments. The major classes of dyes are azo (mono-, di-, tri-, etc.), carbonyl (anthraquinone and indigo derivatives), cyanine, di- and triphenylmethane and phthalocyanine. All these dyes contain chromophore groups, which give rise to color. These chemical structures constitute several cellulosic dye classes, i.e. vat, sulfur, azoic, direct, and reactive dyes as defined in the Colour Index. Three of these dye types involve an oxidation/reduction mechanism, i.e., vat, sulfur and azoic dyes. The purpose of the oxidation/reduction step in these dyeing procedures is to change the dyestuff between an insoluble and a soluble form.

Processing and dyeing procedures are performed in either a batch or continuous mode, with the fabric being contacted by the liquid processing stream in open width or rope form. In continuous methods, a saturator is used to apply chemicals to the fabric, after which the fabric is heated in a chamber where the chemical reaction takes place. A washing section then prepares the fabric for the next processing step. Batch processing generally takes place in one processing bath whereby the fabric is circulated through the bath. After a reaction period, the chemicals are drained, fabric rinsed and the next chemical is applied. Discontinuous pad-batch processing involves a continuous application of processing chemical followed by a dwell period, which, in the case of cold pad-batch, might be one or more days.

Regardless of whether batch, continuous, or discontinuous pad-batch methods are used, bleach clean up, biopolishing and dyeing steps have not heretofore been compatible, due to the broad variation of conditions present in each of the steps. Consequently, it has been necessary to rinse or otherwise treat the fabric or to replace the treating solutions between bleach clean up, biopolishing and dyeing. Thus, there is a need in the art for harmonization of these three steps so that they can be performed in a single bath, whether simultaneously or sequentially, so as to shorten processing time, conserve materials, and reduce the waste stream.

### Summary of the Invention

Therefore, the present invention provides a one-step process for combined biopolishing and beach clean up of treating textile, comprising treating the textile with (i) a system for removing hydrogen peroxide, and (ii) an enzyme system for bio-polishing, wherein the system for removing hydrogen peroxide and the enzyme system for bio-polishing are added simultaneously or sequentially to a single solution.

The process of biopolishing and beach clean up can be further combined with dyeing step and performed in a single solution. A process for combined bleach clean up, biopolishing and dyeing of treating textile, comprising (i) adding into the aqueous solution, an enzyme system for removing hydrogen peroxide, (ii) adding an enzyme system for bio-polishing, wherein step (i) and (ii) take place simultaneously or sequentially in a single solution containing the textile, and (iii) supplementing the aqueous solution with one or more dyestuff and incubating the textile for a sufficient time to achieve dyed textile.

Preferably, the dyestuff is reactive dye. Preferably, the system for removing hydrogen peroxide is the enzyme system comprises catalase, and the enzyme system for bio-polishing comprises celluase and/or protease.

Preferably, the system for removing hydrogen peroxide is chemical reducing agent, and the enzyme system for bio-polishing is celluase and/or protease.

Preferably, the textile is cellulose-containing or cellulosic fabrics, such as cotton, viscose, rayon, ramie, linen, lyocell, or mixtures thereof, or mixtures of any of these fibers together with synthetic fibres or other natural fibers.

Preferably, the cellulase is derived from species of *Humicola, Thermomyces, Bacillus, Trichoderma, Fusarium, Myceliophthora, Phanerochaete, Irpex, Scytalidium, Schizophyllum, Penicillium, Thielavia, Aspergillus,* or *Geotricum, and more preferably* the cellulase is derived from *Thielavia terrestis, Myceliophthora thermophila, Humicola insolens, Trichoderma reesei, Trichoderma harzianum, Aspergillus aculeatus,* and *Aspergillus niger.*

Preferably, the catalase is derived from bacteria such as *Bacillus, Pseudomonas or Streptomyces strain;* yeast such as *Candida, Kluyveromyces, Pichia, Saccharomyces, Yarrowia or Schizosaccharomyces;* fungal such as *Acremonium, Aspergillus, Coprinus, Aureobasidium, Bjerkandera, Humicola, Ceriporiopsis, Coriolus, Cryptococcus, Filibasidium, Fusarium, Magnaporthe, Mucor, Myceliphthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Tramefes or Trichoderma strain;* or animal such as pig liver, beef lever. The present invention further provides an enzyme composition comprising (i) a system for removing hydrogen peroxide, and (ii) an enzyme system for bio-polishing. The system for removing hydrogen peroxide can be catalase, or chemical reducing agent. The chemical reducing agent is preferably sodium hyposulphate, while the enzyme system for biopolishing is preferably cellulase.

When biopolishing and bleach clean up take place simultaneously or sequentially in the same treating solution, compared to traditional processes involving separated biopolishing and bleach clean up, a pH adjusting step and temperature adjusting step are avoided in the present invention. Another advantage of the invention is that process time is saved/reduced as biopolishing and bleach clean up may be carried out simultaneously. The methods and compositions of the present invention of combining bio-polishing and bleaching clean-up in one step thus save time, energy and water. Furthemore, when dyeing step is combined with bleach clean up and biopolishing in the same treating solution, it make the process even more efficient.

### Detailed Description of the Invention

### Textiles

In context of the invention the term "textile" referes to fabrics.

Fabric can be constructed from fibers by weaving, knitting or non-woven operations. Weaving and knitting require yarn as the input whereas the non-woven fabric is the result of random bonding of fibers (paper can be thought of as non-woven). In the present context, the term "fabric" is also intended to include fibers and other types of processed fabrics.

Woven fabric is constructed by weaving 'filling" or weft yarns between wrap yarns stretched in the longitudinal direction on the loom. The wrap yarns must be sized before weaving in order to lubricate and protect them from abrasion at the high speed insertion of the filling yarns during weaving. The filling yarn can be woven through the warp yarns in a "over one - under the next" fashion (plain weave) or by "over one - under two" (twill) or any other myriad of permutations. Strength, texture and pattern are related not only to the type/quality of the yarn but also the type of weave. Generally, dresses, shirts, pants, sheeting's, towels, draperies, etc. are produced from woven fabric.

Knitting is forming a fabric by joining together interlocking loops of yarn. As opposed to weaving which is constructed from two types of yarn and has many "ends", knitted fabric is produced from a single continuous strand of yarn. As with weaving, there are many different ways to loop yarn together and the final fabric properties are dependent both upon the yarn and the type of knit. Underwear, sweaters, socks, sport shirts, sweat shirts, etc. are derived from knit fabrics.

Non-woven fabrics are sheets of fabric made by bonding and/or interlocking fibers and filaments by mechanical, thermal, chemical or solvent mediated processes. The resultant fabric can be in the form of web-like structures, laminates or films. Typical examples are towels, wipes, surgical gowns, fibers for the "environmental friendly" fashion, filter media, bedding, roofing materials, backing for two-dimensional fabrics and many others.

According to the invention, the method of the invention may be applied to any fabric known in the art (woven, knitted, or non-woven). In particular the process of the present invention may be applied to cellulose-containing or cellulosic fabrics, such as cotton, viscose, rayon, ramie, linen, lyocell (e.g. Tencel, produced by Courtaulds Fibers), or mixtures thereof, or mixtures of any of these fibers together with synthetic fibres (e.g., polyester, polyamid, nylon) or other natural fibers such as wool and silk., such as viscose/cotton blends, lyocell/cotton blends, viscose/wool blends, lyocell/wool blends, cotton/wool blends; flax (linen), ramie and other fabrics based on cellulose fibers, including all blends of cellulosic fibers with other fibers such as wool, polyamide, acrylic and polyester fibers, e.g., viscose/cotton/polyester blends, wool/cotton/polyester blends, flax/cotton blends etc. The term "wool," means any commercially useful animal hair product, for example, wool from sheep, camel, rabbit, goat, llama, and known as merino wool, Shetland wool, cashmere wool, alpaca wool, mohair, etc. and includes wool fiber and animal hair. The method of the invention can be used with wool or animal hair material in the form of top, fiber, yarn, or woven or knitted fabric. The enzymatic treatment can also be carried out on loose flock or on fibers made from wool or animal hair material. The treatment can be performed at many different stages of processing. The fabric to be bleached may be dyed or undyed. According to the invention textile may be desized, scoured and/or bleached in aqueous medium in the presence of a fatty acid oxidizing enzyme.

### Enzyme composition

As used in the present invention, an enzyme composition comprises a bio-polishing enzymes system and a hydrogen peroxide removing system, which may be formulated as a liquid (e.g. aqueous), a solid, a gel, a paste or a dry product formulation. Accordingly, the enzymes composition may comprise one or more bio-polishing enzymes, and one or more enzymes for removing hydrogen peroxide or one or more chemical reducing agents for removing hydrogen peroxide.

"Bio-polishing enzymes system" comprises one or more bio-polishing enzyme(s), such as cellullase, protease. "Hydrogen peroxide removing system" comprises one or more enzyme(s) such as catalase, and/or chemical reducing agent(s) for removing hydrogen peroxide, which may also be referred as "Bleach Clean-up system".

### Bio-polishing Enzymes:

The process of the invention comprises cellulase and/or protease treatment of the fabric.

Cellulase is generally used in biopolishing process for textile like cellulosic fabrics, and cellulosic fabrics/synthetic fibres blends, while protease is used in shrinkage-resistance process for natural protein fibers such as wool and silk. Preferably, cellulase together with protease can be used in biopolishing process of blends of cellulosic fibers with other fibers, such as cellulosic fabrics/wool blends, cellulosic fabrics/synthetic fibres/wool blends and etc.

### Cellulase

The term "cellulase" denotes an enzyme that contributes to the hydrolysis of cellulose, such a cellobiohydrolase (abbreviated as "CBH", Enzyme Nomenclature E.C. 3.2.1.91), an endoglucanase (hereinafter abbreviated as "EG", E.C. 3.2.1.4), or a beta-glucosidase (abbreviated as "BG", E.C. 3.2.1.21). Cellulases are classified in a series of enzyme families encompassing endo- and exo- activities as well as cellobiose hydrolyzing capability. The cellulase used in practicing the present invention may be derived from microorganisms which are known to be capable of producing cellulolytic enzymes, such as, e.g., species of *Humicola, Thermomyces, Bacillus, Trichoderma, Fusarium, Myceliophfhora, Phanerochaete, Irpex, Scytalidium, Schizophyllum, Penicillium, Thielavia, Aspergillus,* or *Geotricum,* particularly *Humicola insolens, Fusarium oxysporum,* or *Trichoderma reesei.* Non-limiting examples of suitable cellulases are disclosed in U.S. Patent No. 4,435,307; European patent application No. 0 495 257; PCT Patent Application No. WO91/17244, WO91/17243, WO98/12307; and European Patent Application No. EP-A2-271 004.

The cellulases used in this invention can be monocomponent or multi-components. Monocomponent, i.e. a cellulase which is essentially free from other proteins, in particular other cellulases. Monocomponent enzymes can be prepared economically by recombinant DNA technology, i.e. they can be produced by cloning of a DNA sequence encoding the monocomponent, subsequently transforming a suitable host cell with the DNA sequence and expressing the component in the host. Cellulases of multi-components contain more than one cellulase. Cellulases of muti-components can be blends of two or more cellulases, or can be derived from wild type strain.

The DNA sequence coding for a useful cellulase may for instance be isolated by screening a cDNA library of the microorganism in question and selecting for clones expressing the appropriate enzyme activity (i.e. cellulase activity).

A DNA sequence coding for a homologous enzyme, i.e. an analogous DNA sequence, may be obtainable from other microorganisms. For instance, the DNA sequence may be derived by similarly screening a cDNA library of another fungus, such as a strain of an *Aspergillus sp.,* in particular a strain of *A. aculeatus* or *A. niger,* a strain of *Trichoderma sp.,* in particular a strain of *T. reesei, T. viride, T. longibrachiatum, T. harzianum* or *T. koningii* or a strain of a *Neocallimasfix sp., a Piromyces sp., a Penicillium sp.,* an *Agaricus sp.,* or a *Phanerochaete sp.*

Preferably, the cellulase is derived from or producible by a strain of *Scytalidium (f. Humicola), Fusarium,Myceliophthora,* more preferably derived from or producible by *Scytalidium thermophilum (f. Humicola insolens), Fusarium oxysporum or Myceliophthora themophila,* most preferably from *Humicola insolens, DSM 1800, Fusarium oxysporum, DSM* 2672, or Myceliophthora *themophila, CBS117.65.*

In one embodiment of the invention, the cellulase is an endoglucanase, preferably cellulase from family 45 EG, such as the amino acid sequence of the Thielavia terrestis endoglucanase shown in SEQ ID No. 1 (as described in WO 96/29397) or is an analogue of said endoglucanase which is at least 60% homologous with the sequence shown in SEQ ID No. 1, reacts with an antibody raised against said endoglucanase, and/or is encoded by a DNA sequence which hybridizes with the DNA sequence encoding said endoglucanase. In another embodiment of the invention, the cellulase is an endoglucanase comprising the amino acid sequence of the Humicola insolens endoglucanase shown in SEQ ID No. 2 (as described in WO 91/17243) or is an analogue of said endoglucanase which is at least 60% homologous with the sequence shown in SEQ ID No. 2, reacts with an antibody raised against said endoglucanase, and/or is encoded by a DNA sequence which hybridizes with the DNA sequence encoding said endoglucanase. In a further embodiment of the invention, the celllulases used in the invention are commercially available multi-components cellulase enzyme product such as Cellusoft L, Cellish L (Novozymes A/S, Denmark), Primafast 100, Primafast 200 (Genencor International Inc.), Rocksoft ACE (Dyadic), and Youteer 800 (Youteer Co. Ltd, China).

The host cell which is transformed with the DNA sequence is preferably a eukaryotic cell, in particular a fungal cell such as a yeast or filamentous fungal cell. In particular, the cell may belong to a species of *Aspergillus* or *Trichoderma,* most preferably *Aspergillus oryzae* or *Aspergillus niger.* Fungal cells may be transformed by a process involving protoplast formation and transformation of the protoplast followed by regeneration of the cell wall in a manner known per se. The use of *Aspergillus* as a host microorganism is described in EP 238 023 (Novo Nordisk A/S), the contents of which are hereby incorporated by reference. The host cell may also be a yeast cell, e.g. a strain of *Saccharomyces,* in particular *Saccharomyces cerevisiae, Saccharomyces kluyveri* or *Saccharomyces uvarum,* a strain of *Schizosaccharomyces sp.,* such as *Schizosaccharomyces pombe,* a strain of *Hansenula sp., Pichia sp., Yarrowia sp.* such as *Yan-owia lipolytica,* or *Kluyveromyces sp.* such as *Kluyveromyces lactis.*

In the context, an analogue of the proteins comprises "variant proteins". In some preferred embodiments, variants proteins differ from a parent protein, e.g., a wild-type protein, and one another by a small number of amino acid residues. In the present context, the term "homologous" or "homologous sequence" is intended to indicate an amino acid sequence differing fromanother protein, by one or more amino acid residues, preferably 1, 2, 3, 4, 5, 10, 15, 20, 30, 40, 50 or more amino acid residues. For example, in some embodiments, variant proteins have one to ten difference from the parent protein. The homologous sequence may be one resulting from modification of an amino acid sequence shown in these listings, e.g. involving substitution of one or more amino acid residues at one or more different sites in the amino acid sequence, deletion of one or more amino acid residues at either or both ends of the enzyme or at one or more sites in the amino acid sequence, or insertion of one or more amino acid residues at one or more sites in the amino acid sequence.

However, as will be apparent to the skilled person, amino acid changes are preferably of a minor nature, that is conservative amino acid substitutions that do not significantly affect the folding or activity of the protein, small deletions, typically of one to about 30 amino acids; small amino- or carboxyl-terminal extensions, such as an amino-terminal methionine residue, a small linker peptide of up to about 20-25 residues, or a small extension that facilitates purification, such as a poly-histidine tract, an antigenic epitope or a binding domain. See in general Ford et al., Protein Expression and Purification 2: 95-107, 1991. Examples of conservative substitutions are within the group of basic amino acids (such as arginine, lysine, histidine), acidic amino acids (such as glutamic acid and aspartic acid), polar amino acids (such as glutamine and asparagine), hydrophobic amino acids (such as leucine, isoleucine, valine), aromatic amino acids (such as phenylalanine, tryptophan, tyrosine) and small amino acids (such as glycine, alanine, serine, threonine, methionine).

The modification of the amino acid sequence may suitably be performed by modifying the DNA sequence encoding the enzyme, e.g. by site-directed or by random mutagenesis or a combination of these techniques in accordance with well-known procedures. Alternatively, the homologous sequence may be one of an enzyme derived from another origin than the cellulases corresponding to the amino acid sequences shown in each of the sequence listings shown hereinafter, respectively. Thus, "homologue" may e.g. indicate a polypeptide encoded by DNA which hybridizes to the same probe as the DNA coding for the cellulase with the amino acid sequence in question under certain specified conditions (such as presoaking in 5 x SSC and prehybridising for 1 h at ~40°C in a solution of 20% formamide, 5 x Denhardt's solution, 50 mM sodium phosphate, pH 6.8, and 50 mg of denatured sonicated calf thymus DNA, followed by hybridization in the same solution supplemented with 100 mM ATP for 18 h at ~40°C). The homologous sequence will normally exhibit a degree of homology (in terms of identity) of at least 50%, such as at least 60%, 65%, 70%, 75%, 80%, 85%, 90% or even 95% with the amino acid sequences shown in each of the sequence listings shown hereinafter, respectively.

The homology referred to above is determined as the degree of identity between the two sequences indicating a derivation of the first sequence from the second. The homology may suitably be determined by means of computer programs known in the art such as GAP provided in the GCG program package (Needleman, S.B. and Wunsch, C.D., Journal of Molecular Biology, 48: 443-453, 1970).

### Proteases

Any protease suitable for use in the present invention may be employed.

Suitable proteases include those of animal, vegetable or microbial origin, preferably of microbial origin. Preferably, the protease may be a serine protease or a metalloprotease, more preferably, an alkaline microbial protease or a trypsin-like protease. Examples of proteases include aminopeptidases, including prolyl aminopeptidase (3.4.11.5), X-pro aminopeptidase (3.4.11.9), bacterial leucyl aminopeptidase (3.4.11.10), thermophilic aminopeptidase (3.4.11.12), lysyl aminopeptidase (3.4.11.15), tryptophanyl aminopeptidase (3.4.11.17), and methionyl aminopeptidase (3.4.11.18); serine endopeptidases, including chymotrypsin (3.4.21.1), trypsin (3.4.21.4), cucumisin (3.4.21.25), brachyurin (3.4.21.32), cerevisin (3.4.21.48) and subtilisin (3.4.21.62); cysteine endopeptidases, including papain (3.4.22.2), ficain (3.4.22.3), chymopapain (3.4.22.6), asclepain (3.4.22.7), actinidain (3.4.22.14), caricain (3.4.22.30) and ananain (3.4.22.31); aspartic endopeptidases, including pepsin A (3.4.23.1), Aspergillopepsin I (3.4.23.18), Penicillopepsin (3.4.23.20) and Saccharopepsin (3.4.23.25); and metalloendopeptidases, including Bacillolysin (3.4.24.28).

Commercially available proteases include Alcalase, Savinase, Primase, Duralase, Esperase, Kannase, and Durazym (available from Novozymes A/S), Maxatase, Maxacal, Maxapem, Properase, Purafect, Purafect OxP, FN2, FN3 and FN4 (available from Genencor International Inc.).

Also useful in the present invention are protease variants, such as those disclosed in EP 130,756 (Genentech), EP 214,435 (Henkel), WO 87/04461 (Amgen), WO 87/05050 (Genex), EP 251.446 (Genencor), EP 260.105 (Genencor), Thomas et al., (1985), Nature. 318, p. 375-376, Thomas et al., (1987), J. Mol. Biol., 193, pp. 803-813, Russel et al., (1987), Nature, 328, p. 496-500, WO 88/08028 (Genex), WO 88/08033 (Amgen), WO 89/06279 (Novozymes A/S), WO 91/00345 (Novozymes A/S), EP 525 610 (Solvay) and WO 94/02618 (Gist-Brocades N.V.). The activity of proteases can be determined as described in "Methods of Enzymatic Analysis", third edition, 1984, Verlag Chemie, Weinheim, vol. 5.

### Bleach clean-up system:

The Bleach Clean-up system can be Bleach Clean-up enzyme and/or chemical reducing agent to remove hydrogen peroxide in solution.

### Bleach clean-up Enzymes:

Bleach clean-up enzymes refer to enzymes which can catalyze for the conversion of hydrogen peroxide into water and oxygen, such as catalase (EC 1.11.1.6). Preferred catalases that are suitable for use in a process according to the invention are catalases that is derived from bacteria such as *Bacillus, Pseudomonas or Streptomyces strain;* yeast such as *Candida, Kluyveromyces, Pichia, Saccharomyces, Yarrowia or Schizosaccharomyces;* fungal such as *Acremonium, Scytalidium, Aspergillus, Coprinus, Aureobasidium, Bjerkandera, Humicola, Ceriporiopsis, Coriolus, Cryptococcus, Filibasidium, Fusarium, Magnaporthe, Mucor, Myceliphthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Tramefes or Trichoderma strain;* or animal such as pig liver, beef lever.. Non-limiting examples of suitable catalases are disclosed in WO92/17571, CN1563373, US2003100112-A1, EP1336659-A, US2003074697-A1, US6201167-B1, US6022721-A, EP931831-A, JP11046760-A, WO9317721-A, WO9309219-A, JP1086879-A and/or JP63003788-A. Non-limiting examples are T 100; Oxy-Gone 400 (GCI); Fermcolase 1000 (Mitsubishi Gas Chemical) or Thermocatalase CTL 200 or JH CT 1800 (Mitsubishi Gas Chemical).

In one embodiment of the present invention, the catalase used is derived from *Scytalidium thermophilum* with SEQ ID No:3 (as described in US 5,646,025), or analogue or particularly variant proteins of it.

Depending on the activity of the catalase and the pH of the liquor used to apply the catalase, preferably the amount of catalase used is from 0.001 to 1 g/l, especially about 5 g/l of liquor used to apply the catalase.

### Chemical Reducing Agent

Chemical reducing system refer to any chemical reducing agent(s) for removing hydrogen peroxide by catalyzing the conversion of hydrogen peroxide into water and oxygen. Preferably, the reducing agent can be those widely used in textile industry, like sodium thiosulphate, sodium bisulphite, sodium hydrosulphite and sodium hyposulphate etc.

### Dyestuff

In practicing the present invention, any dyestuff may be used that is compatible with the conditions used for biopolishing.

Conventional dyeing stuffs, comprising one or more of reactive dyes, such as, e.g., C. I. Reactive Red 1, 3, 6, 17, 120, 194, Blue 4, 19, 171 and 182, Black 5, Violet 5; vat dyes, such as, e.g., C. I. Vat Yellow 28, Orange 11 and 15, Blue 6, 16 and 20, Green 1 and 3, 8, Brown 1, Black 9, 27; direct dyes, such as, C. I. Direct Red 81, Yellow 11 and 28, Orange 39, Red 76, Blue 78, 86, 106, 107 and 108, Black 22; sulfur dyes, such as, e.g., C. I. Sulfur Black 1 and 11, Brown 1, Red 10; and azoic dyes, such as, e.g., C. I. Coupling Components 5 and 13 in combination with C. I. Azoic Diazo Components 44 and 45. Such dyes are well known in the art and are described, e.g., in Shore, ed., Cellulosic Dyeing, Society of Dyers and Colorists, Alden Press, 1995; and in Colour Index, Society of Dyers and Colorists and American Association of Textile Chemists and Colorists, Vols. 1-8 Supplements, 1977-1988.

### Additional components:

In some embodiments of the invention, the aqueous solution or wash liquor further comprises other components, including without limitation other enzymes, as well as surfactants, stabilizer, wetting agent, dispersing agents, antifoaming agents, lubricants, builder systems, and the like, or a mixture thereof, that enhance the scouring and/or bleaching processes and/or provide superior effects related to, e.g., strength, resistance to pilling, water absorbency, and dyeability.

The enzymes may be isolated from their cell of origin or may be recombinantly produced, and may be chemically or genetically modified. It will be understood that the amount of enzymatic activity units for each additional enzyme to be used in the methods of the present invention in conjunction with a particular bio-polishing enzyme can be easily determined using conventional assays.

Surfactants suitable for use in practicing the present invention include, without limitation, nonionic, anionic; cationic; and zwitterionic surfactants; which are typically present at a concentration of between about 0.2% to about 15% by weight, preferably from about 1% to about 10% by weight. Anionic surfactants include, without limitation, linear alkylbenzenesulfonate, α-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid, and soap. Non-ionic surfactants include, without limitation, alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, and N-acyl N-alkyl derivatives of glucosamine ("glucamides").

Builder systems include, without limitation, aluminosilicates, silicates, polycarboxylates and fatty acids, materials such as ethylenediamine tetraacetate, and metal ion sequestrants such as aminopolyphosphonates, particularly ethylenediamine tetramethylene phosphonic acid and diethylene triamine pentamethylenephosphonic acid, which are included at a concentration of between about 5% to 80% by weight, preferably between about 5% and about 30% by weight.

Antifoam agents include without limitation silicones (U.S. Patent No. 3,933,672; DC-544 (Dow Corning), which are typically included at a concentration of between about 0.01% and about 1% by weight.

The compositions may also contain soil-suspending agents, soil-releasing agents, optical brighteners, abrasives, and/or bactericides. All of such further components suitable for textile use are well know in the art.

### Process for single bath bleach clean up and biopolishing

The process of the present invention of one-step combined biopolishing and bleach clean up takes place after bleaching step for treating textile.

The manner in which the aqueous solution containing the enzyme system for BioPolishing and Bleach Clean-up system is contacted with the textile will depend upon whether the processing regime is continuous, discontinuous pad-batch or batch. For example, for continuous or discontinuous pad-batch processing, the aqueous enzyme solution is preferably contained in a saturator bath and is applied continuously to the cotton-containing fabric as it travels through the bath, during which process the cotton-containing fabric typically absorbs the processing liquor at an amount of 0.5-1.5 times its weight. In batch operations, the cotton-containing fabric is exposed to the enzyme solution for a period ranging from about 5 minutes to 24 hours at a liquor-to-fabric ratio of 4:1-50:1.

The aqueous solution or wash liquor typically has a pH of between about 4 and about 11. Preferably, the pH of the treating composition is between about 5 and about 10, preferably between about 5 to about 9, and most preferably about 6 to about 7.

In one embodiment, the single-bath method for treating cotton-containing fabric is carried out at a pH below 9, more preferably, below 8, and even more preferably below 7.

The temperature at which the combined Bleach Clean-up and BioPolishing processes are carried out will depend on the process used. In the case of cold pad batch process, the temperature for the combined bleaching clean-up and bio-polishing processes is preferably between about 15°C and about 65°C, and most preferably between about 25°C and about 60°C. For continuous and other batch processes, the temperature for carrying out combined bleaching clean-up and bio-polishing processes is preferably between about 35°C and about 75°C, and most preferably between about 45°C and about 65°C.

The enzyme for biopolishing is generally added in an amount which is effective to generate enough biopolishing effect of the textile material. The enzyme(s) may preferably be dosed in an amount of 0.01 mg protein/l to 1g protein/l of the total liquor, more preferably from 0.1 mg protein/I to 300mg protein/I, most preferably from 1 mg protein/l to 150mg protein/l.

The enzyme for bleach clean up is generally added in an amount which is effective to remove hydrogen peroxid. The enzyme(s) may preferably be dosed in an amount about from 0.001mg protein/l to 1 g protein/I of the total liquor, preferably 0.01mg protein/I to 100mg protein/I, most preferably 0.15mg protein/l to about 1 mg protein/I.

The chemical reductive agent for bleach clean up may be dosed in an amount of from about 0.01 g/I to about 10 g/l of the total liquor, more preferably, from about 0.1 g/I to about 3 g/l, most preferably, from about 0.5 g/I to about 1 g/l.

It will be understood that the optimum dosage and concentration of the enzymes, bleach compounds, bleach stabilizers, the volume of the aqueous solution or wash liquor, and the pH and temperature will vary, depending on: (i) the nature of the fiber, i.e., crude fiber, yarn, or textile; (ii) the particular enzyme(s) used, and the specific activity of the enzyme; (iii) the conditions of temperature, pH, time, etc., at which the processing occurs; (iv) the presence of other components in the wash liquor; and (v) the type of processing regime used, i.e., continuous, discontinuous pad-batch, or batch. The optimization of the process conditions can be determined using routine experimentation, such as, by establishing a matrix of conditions and testing different points in the matrix. For example, the amount of enzyme, the temperature at which the contacting occurs, and the total time of processing can be varied, after which the resulting cellulosic materials or textile is evaluated for (a) pilling result; and (b) residual H₂O₂ ppm.

Further, according to the present invention, bleach clean up, biopolishing and dyeing steps can be achieved in a single-bath. In one embodiment, (i) adding catalase and cellulase for bleach clean up and biopolishing, a first incubation is performed for a sufficient time to remove the residual hydrogen peroxide, (ii) the wash liquor is then supplemented with dyestuffs and a second incubation is performed for a sufficient time and under appropriate conditions to achieve effective dyeing. Preferably, after adding catalase and cellulase the incubation time for step (i) is no less than 5 minutes, so most of the hydrogen peroxide has been removed. More preferably, the incubation time for step (i) is no less than 10 minutes, even more preferably no less than 15 minutes and no more than 2 hours. Most preferably, the incubation time for step (i) is 10-20 minutes, so as to remove almost all of the hydrogen peroxide and shorten the whole process time.

It will be understood that before or during step (ii) of dyeing, it further comprise adjusting one or more properties of the composition of the wash liquor, such as ionic strength by adding salt. And the conditions of incubation in dyeing step may also differ with respect to temperature, agitation, pH, time, and the like. The performances of biopolishing with enzymes still continue during incubation of dyeing process, so that the whole process time will be shortened.

Preferably, the dyestuff used in present invention is reactive dye, which works well with cellulase disclosed herein.

The salt added before or after the adding of dyestuff preferably is NaCl or Na₂SO₄ which take control of the uptake of the dyestuff on the fiber. The concentration of salt in the wash liquor depends on the concentration of dyestuff and what kind of dyestuff used. Normally, the higher concentration of dyestuff is required, the higher salt concentration is needed. Typically, the dyestuff in the wash liquor is 0.001-15%, preferably 0.001-9% of the weight of fabric. The concentration of salt (eg. Na₂SO₄) is 10-100 g/l.

The wash liquor in dyeing step preferably has a pH between about 5 and about 8, most preferably between about 6 and about 7.

The temperature at which the subsequent dyeing is carried out may be between about 30°C and about 100°C, preferably between about 40°C and about 90°C, and most preferably between about 40°C and about 80°C.

It will be understood that in most cases, the pH and temperature in bleach clean up and biopolishing step are suitable for the dyeing step, especially for reactive dye. So, there is no need to adjust pH and temperature during dyeing.

The following are intended as non-limiting illustrations of the present invention.

### EXAMPLES

### Materials & Methods

### Enzymes

- Catalase A: Catalase with SEQ ID NO.:3
- Catalase T100 (Genencor international Inc)
- Catalase ASC 200(Mitsubishi)
- Neutral Cellulase B with SEQ ID NO.:2
- Neutral Cellulase A with SEQ ID NO.:1
- Acid Cellulase C : CELLUSOFT L^{™} (NovozymesA/S)
- Primafast Luna RL (Genencor international Inc)
- Indiage Max L (Genencor international Inc)

### Fabric

- 460-60 bleached Interlock Knits (Testfabrics, Inc.)
- Dyed fabric (Garment bought from Carrfour)

### Dyestuff

- Reactive RED BF-3B (Arugs textile auxiliary CO.LTD)
- Reactive Yellow BF-3R (Arugs textile auxiliary CO.LTD)
- Reactive TQ blue G (Arugs textile auxiliary CO.LTD)

### Buffer

2.716g of potassium dihydrogen phosphate and 0.201g sodium hydroxide are dissolved in 1L de-ionized water.
1g/l NAAC adjust with HAC to pH 5

### Methods:

### Catalase activity (KCIU Assay)

When used according to the present invention the activity of catalase may be measured in KCIU. One CIU will decompose one µmole of H₂O₂ per minute at pH 7.0, 25°C while the H₂O₂ concentration decreases from 10.3 to 9.2 µmoles per ml reaction mixture.

Catalase catalyzes the first order reaction:

2 H₂O₂→ 2H₂O + O₂

The degradation of hydrogen peroxide is monitored using spectrophotometry at 240 nm. The length of time for a specified decrease in absorbance, at a specified H₂O₂ concentration, is a measure of the catalase activity.

### Reaction

| | |
|---|---|
| Enzyme concentration | approx.100 CIU/ml |
| Substrate Concentration | 10.3 mM H₂O₂ |
| pH | 7.0 ± 0.05 |
| Buffer | 50 mM phosphate |
| Temperature | 25 C |

### Detection

| | |
|---|---|
| Wave length | 240 nm |
| Absorbance interval | 0.450-0.400 |
| Time interval | 0.267 - 0.400 minutes (16-24 seconds) |

A folder describing this analytical method in more detail is available upon request to Novozymes A/S, Denmark, which folder is hereby included by reference.

### Cellulase activity (ECU)

Cellulase samples are incubated with a carboxymethyl cellulose (CMC) substrate. Degradation of the substrate leads to a reduction in viscosity, which is measured using a vibrating-spindle viscometer. The reduction in viscosity is pro-portional to the endo-cellulase activity.

Endo-cellulase activity in ECU is measured relative to a Novozymes A/S enzyme standard.

| **Reaction conditions** | |
|---|---|
| Temperature | 40°C |
| pH | 7.5 |
| Substrate concentration | 3.11 %(m/V) |
| Incubation time | 30 min |
| Enzyme concentration | 0.097 -0.181 ECU/ml |

A folder describing this analytical method in more detail is available upon request to Novozymes A/S, Denmark, which folder is hereby included by reference.

### Cellulase activity (EGU)

Cellulase samples are incubated with carboxymethyl cellulose (CMC) substrate. Degradation of the substrate leads to a reduction in viscosity, which is measured using a vibrating-spindle viscometer. The reduction in viscosity is proportional to the endo-glucanase activity.

| **Reaction Conditions** | |
|---|---|
| Temperature | 40°C |
| pH | 6.0 |
| Substrate concentration | 3.11 % (w/v) |
| Enzyme concentration | 0.01-0.02 EGU/mL |
| Reaction time | 30 minutes |

A folder describing this analytical method in more detail is available upon request to Novozymes A/S, Denmark, which folder is hereby included by reference.

### Bleach clean up (Peroxide Test-strip)

Peroxidase transfers peroxide oxygen to an organic redox indicator. This produces a blue oxidation product. This peroxide concentration is measured semi-quantitatively by visual comparison of the reaction zone of the analytical test strip with the fields of a colour scale. The hydrogen peroxide is determined by immersing the reaction zone of the analytical test strip in the measurement sample for 1 second. Allow excess liquid to run off via the long edge of strip onto an absorbent paper towel and after 15 sec. determine with which colour field on the label on the colour of the reaction zone coincides most exactly. Read off the corresponding result in mg/l H₂O₂ or if necessary, estimate an intermediate value.

### Pilling test

### Martindale

The principle of the pilling test is that two specimens of a test fabric are rubbed against each other in a continuously changing pattern, which ensures that the surface fibers of the specimens are flexed in every direction. After a predetermined number of rubbing cycles, the wear resistance of the specimens are visually evaluated by comparing to EMPA photo scale standards. There are five grades of the pilling results
Note 5 : No pilling
Note 4 : Slight Pilling
Note 3 : Moderate Pilling
Note 2 : Distinct Pilling
Note 1 : Heavy Pilling
1/2 notes are allowed

The testing method refers to GB/T 4802.2-1997

### The ICI Pilling Tester

The ICI Pilling Tester is the most applicable test to use on most types of fabric, both woven and knitted, and its test method runs in accordance with BS EN ISO 12945-1 ( amongst other standards). The Pilling Tester consists of 2 boxes, each being lined with a metal plate supporting 3.2mm thick buffed-finish cork jointing material. The tester features an automatic counter which stops the machine after any predetermined number of revolutions.

Assessment of the pilling standard is visual, using either the standard photographs or the Rating Scheme. The tested specimens are mounted on card and then rated against the standard photos or against an untested sample.

| Rating | Description | Points to be taken into consideration during assessment |
|---|---|---|
| 5 | No change | No visual change |
| 4 | Slight change | Slight surface fuzzing |
| 3 | Moderate change | Test specimen may exhibit moderate fuzzing and/or isolated fully formed pills |
| 2 | Significant change | Distinct fuzzing and/or pilling |
| 1 | Severe change | Dense fuzzing and/or pilling which covers the specimen |

### Protein Content

BCA kit (available from PIERCE) was used for detecting the protein content in the enzyme product.

### Example 1

### Combining Bio-polishing and Bleach clean up into one process conducted with a Laundry-meter

A 100% 460-60 bleached interlock cotton fabric was purchased from Test Fabrics. Fabric swatches were cut to about 5g each.

One buffer at p H 6.5 was used for this study. 2.716g of potassium dihydrogen phosphate and 0.201 g sodium hydroxide were dissolved in 1 L de-ionized water. The process was conducted with a Laundry-meter. The beaker was filled with 100ml buffer and two pieces of pre-cut fabric.
1) Main washing: The beaker was filled with 100ml buffer. Some hydrogen peroxide was added to each beaker with 20 steel balls as specified. In the meanwhile, a neutral Cellulase A was dosed to a concentration and catalase A was added in then temperature was raised to 55°C and kept for 60min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure the weight loss and pilling result on the treated fabrics.

The results of the test are shown in Table 1.

**Table 1**

| H₂O₂ At beginning | Catalase A (mg protein /l) | Neutral Cellulase A (mg protein /l) | Residual H₂O₂ after treatment | Pilling result |
|---|---|---|---|---|
| 0 | 0 | 0 | 0 | 1.5-2 |
| 0 | 0 | 7 | 0 | 4-4.5 |
| 100ppm | 0 | 7 | >25ppm | 4-4.5 |
| 0 | 0.34 | 7 | 0 | 4.5 |
| 100ppm | 0.34 | 0 | 0 | 1.5-2 |
| 100ppm | 0.34 | 7 | 0 | 4.5 |
| 350ppm | 0.34 | 4.2 | 0 | 4-4.5 |

As can be seen from the table, the fabrics treated in the combined process of the invention with a combination of catalase and cellulase have less pilling, and no detectable Residual H₂O₂ in the solution.

### Example 2

### Combining Bio-polishing and Bleach clean up into one process conducted with a wascator

A 100% 460-60 bleached interlock cotton fabric was purchased from Test Fabrics. The weight of the fabric swatches was 1 kg.

One buffer at pH 6.5 was used for this study. 2.716g of potassium dihydrogen phosphate and 0.201 g sodium hydroxide were dissolved in 1L de-ionized water. The process was conducted with a wascator. 1 kg fabric was put in the wascator at the beginning of the process.
1) Main washing: The wascator was filled with 10L water. The temperature was raised to 55°C. In the mean while, 36g of K₂HPO₄ and 14.5g KH₂PO₄ were added in to adjust the pH to 6.5. Some hydrogen peroxide was added to the wascator as specified. A neutral Cellulase was dosed to a concentration and catalase was added in and kept for 60min.
2) Check the residual Hydroperoxide with peroxide strip after 20min.
3) Inactivation: After main washing, drained and re-fill the wascator and added the 1g/l Na₂CO₃ in the wascator then raised the temperature to 80°C and run for 10min, drained.
4) Cold rinse: Filled in cold water and rinsed for 10min
5) Spin off the water on the fabrics and tumble dryer.
6) Measure the pilling result on the treated fabrics.

The result of the testing are shown in Table 2

**Table 2**

| H₂O₂ At beginning | Catalase A (mg protein /l) | Neutral Cellulase A (mg protein /l) | Residual H₂O₂ after treatment for 20min | Pilling result |
|---|---|---|---|---|
| 100ppm | 0.34 | 7 | 0 | 4.5 |

As can be seen from the table, the fabrics treated in the combined process of the invention with a combination of catalase A and neutral cellulase A have less pilling, and no detectable Residual H₂O₂ in the solution.

### Example 3

### Combining Bio-polishing and Bleach clean up into one process conducted on dyed fabric with a Laundrymeter

A 100% dyed cotton fabric was purchased. Fabric swatches were cut to about 10g each.

One buffer at pH 6.5 was used for this study. 2.716g of potassium dihydrogen phosphate and 0.201g sodium hydroxide were dissolved in 1L de-ionized water. The process was conducted with a Laundry-meter. The beaker was filled with 100ml buffer and one piece of pre-cut fabric.
1) Main washing: The beaker was filled with 100ml buffer. Some hydrogen peroxide was added to each beaker with 20 steel balls as specified. In the meanwhile, a neutral Cellulase A was dosed to a concentration and catalase A was added in then temperature was raised to 55°C and kept for 20min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure pilling result on the treated fabrics.

The results of the test are shown in Table 3.

**Table 3**

| H₂O₂ At beginning | Catalase A (mg protein /l) | Neutral Cellulase A (mg protein /l) | Residual H₂O₂ after treatment | Pilling result |
|---|---|---|---|---|
| 0 | 0 | 0 | 0 | 2-2.5 |
| 350ppm | 0 | 0 | >25ppm | 2-2.5 |
| 0 | 0 | 4.2 | 0 | 4 |
| 350ppm | 0 | 4.2 | >25ppm | 3.85 |
| 350ppm | 0.34 | 4.2 | 0 | 4 |
| 0 | 0 | 21 | 0 | 4.5-5 |
| 350ppm | 0 | 21 | >25ppm | 4.5-5 |
| 350ppm | 0.34 | 21 | 0 | 4.5-5 |

As can be seen from the above table, the fabrics treated in the combined process of the invention with a combination of catalase and cellulase have less pilling, and no detectable residual H₂O₂ in the solution. The present invention shows more advantageous effect than those using catalase or cellulase alone.

### Example 4

### Combining Bio-polishing and Bleach clean up into one process with an acid cellulase

A 100% 460-60 bleached interlock cotton fabric was purchased from Test Fabrics. Fabric swatches were cut to about 5g each.

One buffer at pH 5 was used for this study. 1g of sodium acetate were dissolved in 1 L de-ionized water and adjust the pH to 5 with acetic acid. The process was conducted with a Laundry-meter. The beaker was filled with 100ml buffer and two pieces of pre-cut fabric.
1) Main washing: The beaker was filled with 100ml buffer. Some hydrogen peroxide was added to each beaker with 20 steel balls as specified. In the meanwhile, an acid Cellulase C was dosed to a concentration and catalase A was added in then temperature was raised to 55°C and kept for 60min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure the weight loss and pilling result on the treated fabrics.

The results of the test are shown in Table 4.

**Table 4**

| H₂O₂ At beginning | Catalase A (mg protein /l) | Acid Cellulase C (mg protein /l) | Residual H₂O₂ after treatment | Pilling result |
|---|---|---|---|---|
| 0 | 0 | 0 | 0 | 1.5-2 |
| 0 | 0 | 120 | 0 | 4-4.5 |
| 100ppm | 0.34 | 120 | 0 | 3.5-4 |

### Example 5

### Combining Bio-polishing and Bleach clean up into one process with Primafast luna RL

The procedures were the same as described in Example 4 whilst the cellulase used was Primafast luna RL and the catalse used was Catalase A or Catalase T100, ASC 200 individually. The result of the test was shown in Table 5.

### Example 6

### Combining Bio-polishing and Bleach clean up into one process with IndiAge Max L

The procedures were the same as described in Example 4 whilst the cellulase used was Primafast luna RL and the catalse used was Catalase A or Catalase T100, ASC 200 individually. The result of the test was shown in Table 5.

### Example 7

### Combining Bio-polishing and Bleach clean up into one process with Neutral cellulase A

The procedures were the same as described in Example 1 whilst the Catalase used was Catalase T 100 or ASC 200. The result of the test was shown in Table 5.

### Example 8

### Combining Bio-polishing and Bleach clean up into one process with Acid cellulase C

The procedures were the same as described in Example 4 whilst the Catalase used was Catalase T 100 or ASC 200. The result of the test was shown in Table 5

**Table 5**

| H₂O₂ At beginning | Cellulase | Dosage (mg protein /l) | Catalase | Dosage (mg protein /l) | Pilling result | Residual H₂O₂ after treatment |
|---|---|---|---|---|---|---|
| / | Primafast luna RL | 41.88 | / | / | 3.85 | 0 |
| 350ppm | Primafast luna RL | 41.88 | Catalase A | 0.34 | 4-4.5 | 0 |
| 350ppm | Primafast luna RL | 41.88 | Catalase T 100 | 1 | 3.85 | 0 |
| 350ppm | Primafast luna RL | 41.88 | ASC 200 | 0.58 | 3.85 | 0 |
| / | IndiAge Max L | 69 | / | / | 4.5-5 | 0 |
| 350ppm | IndiAge Max L | 69 | Catalase A | 0.34 | 4-4.5 | 0 |
| 350ppm | Indiage Max L | 69 | Catalase T 100 | 1 | 4-4.5 | 0 |
| 350ppm | IndiAqe Max L | 69 | ASC 200 | 0.58 | 4-4.5 | 0 |
| / | Neutral cellulase A | 1.75 | / | / | 4-4.5 | 0 |
| 350ppm | Neutral cellulase A | 1.75 | Catalase T 100 | 1 | 3.5-4 | 0 |
| 350ppm | Neutral cellulase A | 1.75 | ASC 200 | 0.58 | 4-4.5 | 0 |
| / | Acid cellulase C | 120 | / | / | 3.5-4 | 0 |
| 350ppm | Acid cellulase C | 120 | Catalase T 100 | 1 | 3.5-4 | 0 |
| 350ppm | Acid cellulase C | 120 | ASC 200 | 0.58 | 3.5-4 | 0 |

### Example 9

### Combining Biopolishina and Bleach clean up into one process conducted with a Laundry-meter at lower temp

A 100% 460-60 bleached interlock cotton fabric was purchased from Test Fabrics. Fabric swatches were cut to about 5g each.

One buffer at pH 6.5 was used for this study. 2.716g of potassium dihydrogen phosphate and 0.201 g sodium hydroxide were dissolved in 1 L de-ionized water.The process was conducted with a Laundry-meter. The beaker was filled with 100ml buffer and two pieces of pre-cut fabric.
1) Main washing: The beaker was filled with 100ml buffer. Some hydrogen peroxide was added to each beaker with 20 steel balls as specified. In the meanwhile, a neutral Cellulase B was dosed to a concentration and catalase A was added in then temperature was raised to 30°C and kept for 60min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure the weight loss and pilling result on the treated fabrics.

**Table 6**

| H₂O₂ At beginning | Cellulase | Dosage (mg protein /l) | Catalase | Dosage (mg protein /l) | Pilling result | Residual H₂O₂ after treatment |
|---|---|---|---|---|---|---|
| / | / | / | / | / | 1.5-2 | 0 |
| / | Neutral cellualse B | 6 | / | / | 4 | 0 |
| 350ppm | Neutral cellualse B | 6 | Catalase A | 0.34 | 4 | 0 |
| / | Neutral cellualse B | 12 | / | / | 4-4.5 | 0 |
| 350ppm | Neutral cellualse B | 12 | Catalase A | 0.34 | 4.5 | 0 |
| 350ppm | Neutral cellualse A | 7 | Catalase A | 0.34 | 4 | 0 |

### Example 10

### Combining Biopolishing and Bleach clean up with reducing agent into one process conducted with a Laundry-meter

A 100% 460-60 bleached interlock cotton fabric was purchased from Test Fabrics. Fabric swatches were cut to about 5g each.

One buffer at p H 6.5 was used for this study. 2.716g of potassium dihydrogen phosphate and 0.201 g sodium hydroxide were dissolved in 1 L de-ionized water. The process was conducted with a Laundry-meter. The beaker was filled with 100ml buffer and two pieces of pre-cut fabric.
1) Main washing: The beaker was filled with 100ml buffer. Some hydrogen peroxide was added to each beaker with 20 steel balls as specified. In the meanwhile, a neutral Cellulase B was dosed to a concentration and reducing agent (sodium hyposulphate: abbreviated as Hypo) was added in then temperature was raised to 55°C and kept for 60min.
2) Check the residual Hydroperoxide with peroxide strip.
3) Inactivation: After the checking, add the 1g/l Na₂CO₃ in the machine/beaker then raised the temperature to 80°C and run for 10min, drained.
4) Cold rinse: Filled in cold water and rinsed for 10min
5) Spin off the water on the fabrics and tumble dryer.
6) Measure the weight loss and pilling result on the treated fabrics.

The results of the test are shown in Table 7.

**Table 7**

| H₂O₂ At beginning | Enzyme | Dosage (mg protein /l) | Reducing agent | Weight loss% | Residual H₂O₂ | piling result |
|---|---|---|---|---|---|---|
| / | | blank | / | -0.6 | 0 | 1.625 |
| H₂O₂ 350PPM | Neutral Cellulase B | 3 | / | 2.9 | >25ppm | 4.875 |
| H₂O₂ 350PPM | Neutral Cellulase B | 6 | / | 4.3 | >25ppm | 5 |
| H₂O₂ 350PPM | Neutral Cellulase B | 12 | / | 6.5 | >25ppm | 5 |
| H₂O₂ 100PPM | Neutral Cellulase B | 3 | 1g/l Hypo | 3.4 | 0 | 5 |
| H₂O₂ 100PPM | Neutral Cellulase B | 6 | 1g/l Hypo | 4.9 | 0 | 5 |
| H₂O₂ 100PPM | Neutral Cellulase B | 12 | 1g/l Hypo | 6.6 | 0 | 5 |

### Example 11

### One bath Biopolishing, Bleach clean up and dyeing conducted with a Laundry-meter

A 100% 460-60 bleached interlock cotton fabric was purchased from Test Fabrics. Fabric swatches were cut to about 5g each.

**Table 8: Dyeing recipe**

| Sample No | Sample 1 | Sample 2 | Sample 3 | Sample 4 | Sample 5 | Sample 6 |
|---|---|---|---|---|---|---|
| Chemicals used | Dosage | | | | | |
| H₂O₂ | 300ppm | / | 300ppm | / | 300ppm | / |
| Neutral Cellulase A (protein/L) | 7mg/L | / | 7mg/L | / | 7mg/L | / |
| Catalase A (protein/L) | 0.34mg/l | | 0.34mg/l | | 0.34mg/l | |
| Reactive RED BF-3B | 1 % owf | 1 % owf | 2% owf | 2% owf | 3 % owf | 3% owf |
| Reactive Yellow BF-3R | 1 % owf | 1 % owf | 2 % owf | 2% owf | 3% owf | 3 % owf |
| Reactive TQ Blue G | 1% owf | 1 % owf | 2% owf | 2% owf | 3% owf | 3% owf |
| Na₂SO₄ | 40g/l | 40g/l | 60g/l | 60g/l | 80g/l | 80g/l |
| Na₂CO₃ | 15g/l | 15g/l | 20g/l | 20g/l | 20g/l | 20g/l |

| | | | | | | |
|---|---|---|---|---|---|---|
| Owf: of the weight of fabric | | | | | | |

A water solution at pH 6.5 was used for this study. Acetic acid was dissolved in 1 L de-ionized water to adjust pH to 6.5. The process was conducted with a Laundry-meter. The beaker was filled with 100ml solution and two pieces of pre-cut fabric.
1) Single-bath bleach clean up, bio-polishing and dyeing process:
   The beaker was filled with 100ml water solution at pH 6.5 adjusted by acetic acid. Some hydrogen peroxide was added to each beaker with 20 steel balls as specified. In the meanwhile, a neutral Cellulase A was dosed to a concentration and catalase A was added, following that certain amount of sodium sulfate was added in, then temperature was raised to 60°C and kept for 15min. Check the residual Hydroperoxide with peroxide strip.
2) Adding dyestuff and sodium carbonate: After the checking, add certain concentration of dyestuffs (three kinds of dyestuff from Argus) in the machine/beaker and run for 45min, then dosed certain amount sodium carbonate and run for 45min/60min. Drained.
3) Soaping: two steps of soaping process were conducted following the dyeing process. Filled in cold water and added 1g/l soaping agent Dekol SNS(BASF) and raised temperature to 90°C for 10min. Drained. Filled in cold water and added 1g/l soaping agent Dekol SNS (BASF) and raised temperature to 90°C for 10min. Drained.
4) Hot rinse: Filled in cold water and raised temperature to 70°C for 15min.
5) Cold rinse: Filled in cold water and rinsed for 10min.
6) Spin off the water on the fabrics and tumble dryer.
7) Measure the pilling result on the treated fabrics.

The results of the test are shown in Table 9.

**Table 9**

| Sample No | Sample 1 | Sample 2 | Sample 3 | Sample 4 | Sample 5 | Sample 6 |
|---|---|---|---|---|---|---|
| Residual H₂O₂ before adding dyestuff | 0 | n/a | 0 | n/a | 0 | n/a |
| Pilling | 4.5 | 1.5 | 4.25 | 1.5 | 4.25 | 1.5 |

As can be seen from the above table, the fabrics treated in the one bath bleach clean up, bio-polishing and dyeing process of the invention with a combination of catalase and cellulase have less pilling and largely shorten the process time compared to the separated process.

All patents, patent applications, and literature references referred to herein are hereby incorporated by reference in their entirety. Many variations of the present invention will suggest themselves to those skilled in the art in light of the above detailed description. Such obvious variations are within the full-intended scope of the appended claims.

The present invention is also described with reference to the following numbered paragraphs:
1. A one-step process for combined biopolishing and bleach clean up of treating textile, comprising the step of treating the textile with (i) a system for removing hydrogen peroxide, and (ii) an enzyme system for bio-polishing, wherein the system for removing hydrogen peroxide and the enzyme system for bio-polishing are added simultaneously or sequentially to a single solution containing the textile.
2. The process of paragraph 1, wherein the system for removing hydrogen peroxide comprises catalase or chemical reducing agent.
3. The process of paragraph 1, wherein the textile comprises cellulose-containing or cellulosic fabrics.
4. The process of any of paragraphs 1 -3, wherein the enzyme system for biopolishing comprises cellulase.
5. The process of any of the preceding paragraphs, wherein the textile comprises natural protein fabric, such as wool and silk.
6. The process of paragraph 5, wherein the enzyme system for biopolishing comprises protease.
7. The process of any of the preceding paragraphs, wherein the cellulase is derived from species of *Humicola, Thermomyces, Bacillus, Trichoderma, Fusarium, Myceliophthora, Phanerochaete, Irpex, Scytalidium, Schizophyllum, Penicillium, Thielavia, Aspergillus,* or *Geotricum.*
8. The process of paragraph 7, wherein the cellulase is derived from *Thielavia terrestis, Myceliophthora thermophila, Humicola insolens, Trichoderma reesei, Trichoderma harzianum, Aspergillus aculeatus,* and *Aspergillus niger.*
9. The process of paragraphs 4 or 7, wherein the cellulase is family 45 cellulase.
10. The process of paragraph 9, wherein the cellulase is selected from the group consisting of a polypeptide comprising the sequence of SEQ ID NO: 1 or SEQ ID NO. 2, or a variant thereof.
11. The process of paragraph 2, wherein the catalase is derived from bacterial, yeast, fungi, or animal.
12. The process of paragraph 11 , wherein the catalase is derived from bacteria such as *Bacillus, Pseudomonas or Streptomyces strain;* yeast such as *Candida, Kluyveromyces, Pichia, Saccharomyces, Yarrowia or Schizosaccharomyces;* fungal such as *Acremonium, Scytalidium, Aspergillus, Coprinus, Aureobasidium, Bjerkandera, Humicola, Ceriporiopsis, Coriolus, Cryptococcus, Filibasidium, Fusarium, Magnaporthe, Mucor, Myceliphthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, Micrococcus or Trichoderma strain;* or animal such as pig liver, beef lever.
13. The process of paragraph 12, wherein the catalase is *Scytalidium thermophilum* catalase of SEQ ID No:3, or a variant thereof.
14. The process of paragraph 2, wherein the reducing agent is sodium hyposulphate.
15. The process of any of the preceding paragraphs, wherein the concentration of catalase is in the range of from 0.001mg protein/I to 1g protein/I of the total liquor, preferably 0.01mg protein/I to 100mg protein/I, most preferably 0.15mg protein/I to about 1 mg protein/I.
16. The process of any of the preceding paragraphs, wherein the concentration of chemical reducing agent is in the range of from 0.01 g/l to 10 g/I of the total liquor, preferably 0.1 g/l to 3 g/l, most preferably 0.5 g/l to 1 g/l.
17. The process of any of the preceding paragraphs, wherein the enzyme concentration for biopolishing is in the range of from 0.01 mg protein/l to 1g protein/l of the total liquor, more preferably from 0.1 mg protein/l to 300mg protein/l, most preferably from 1 mg protein/l to 150mg protein/l.
18. The process of any of the preceding paragraphs, wherein the pH of the solution is between about 4 and about 12, preferably between about 6 and about 8.
19. The process of any of the preceding paragraphs, wherein the process is carried out at a temperature between 20 degree Celsius to 80 degree Celsius, preferably between 50 degree Celsius to 60 degree Celsius.
20. A one-step process for combined bleach clean up, biopolishing and dyeing of treating textile in a single bath, comprising
   i) adding into the aqueous solution an enzyme system for removing hydrogen peroxide,
   ii) adding an enzyme system for bio-polishing, wherein step (i) and (ii) take place simultaneously or sequentially in a single solution containing the textile, and
   iii) supplementing the aqueous solution with one or more dyestuff and incubating the textile for a sufficient time to achieve dyed textile.
21. The process of paragraph 20, wherein the enzyme system for removing hydrogen peroxide comprises catalase.
22. The process of paragraph 20, wherein the enzyme system for biopolishing comprises cellulase.
23. The process of paragraph 20, wherein after step (i) of adding enzyme system for removing hydrogen peroxide, the incubation time is no less than 5 minutes.
24. The process of paragraph 20, wherein the dyestuff is reactive dye.
25. An enzyme composition comprising (i) a system for removing hydrogen peroxide, and (ii) an enzyme system for bio-polishing.
26. The enzyme composition of paragraph 25, wherein the system for removing hydrogen peroxide comprises catalase or chemical reducing agent.
27. The enzyme composition of paragraph 25 or 26, wherein the enzyme system for biopolishing comprises cellulase and/or protease.
28. The enzyme composition of any one of paragraphs 25-27, wherein the enzyme composition comprises one or more additional components selected from surfactants, stabilizer, wetting agent, dispersing agents, antifoaming agents, lubricants, and builder system, or a mixture thereof.
29. The enzyme composition of paragraph 26, wherein the catalase is derived from bacterial, yeast, fungi, or animal.
30. The enzyme composition of paragraph 29, wherein the catalase is derived from *Scytalidium.*
31. The enzyme composition of paragraph 27, wherein the cellulase is family 45 cellulase.
32. The enzyme composition of paragraph 26, wherein the reducing agent is sodium hyposulphate.

## Claims

1. An enzyme composition suitable for use in a one-step textile treatment process for combined bio-polishing and hydrogen peroxide bleach clean up after a hydrogen peroxide textile bleaching step, comprising (i) a catalase and/or chemical reducing agent, selected among sodium thiosulphate, sodium bisulphite, sodium hydrosulphite and sodium hyposulphate, in combination with (ii) an enzyme system comprising cellulase for bio-polishing.

2. An enzyme composition of claim 1, wherein the enzyme system for biopolishing comprises cellulase and protease.

3. An enzyme composition of claims 1 or 2, wherein the enzyme system for biopolishing comprises an endoglucanase.

4. An enzyme composition of any of claims 1-3, wherein the cellulase is derived from species of *Humicola, Thermomyces, Bacillus, Trichoderma, Fusarium, Myceliophthora, Phanerochaete, Irpex, Scytalidium, Schizophyllum, Penicillium, Thielavia, Aspergillus,* or *Geotricum.*

5. An enzyme composition of claim 4, wherein the cellulase is derived from *Thielavia terrestis, Myceliophthora thermophila, Humicola insolens, Trichoderma reesei, Trichoderma harzianum, Aspergillus aculeatus,* or *Aspergillus niger.*

6. An enzyme composition of any of claims 1-5, wherein the cellulase is family 45 cellulase.

7. An enzyme composition of claim 6, wherein the family 45 cellulase is selected from the group consisting of a polypeptide comprising the sequence of SEQ ID NO: 1 or SEQ ID NO. 2, or a variant thereof which has at least 70% identity to SEQ ID NO: 1 or SEQ ID NO: 2.

8. An enzyme composition of any of the preceding claims, wherein the catalase is derived from bacterial, yeast, fungi, or animal.

9. An enzyme composition of claim 8, wherein the catalase is derived from bacteria such as *Bacillus, Pseudomonas or Streptomyces strain;* yeast such as *Candida, Kluyveromyces, Pichia, Saccharomyces, Yarrowia or Schizosaccharomyces;* fungal such as *Acremonium, Scytalidium, Aspergillus, Coprinus, Aureobasidium, Bjerkandera, Humicola, Ceriporiopsis, Coriolus, Cryptococcus, Filibasidium, Fusarium, Magnaporthe, Mucor, Myceliphthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, Micrococcus or Trichoderma strain;* or animal such as porcine liver, bovine liver.

10. An enzyme composition of claim 9, wherein the catalase is *Scytalidium thermophilum* catalase of SEQ ID No:3.

11. An enzyme composition of any of the preceding claims, wherein the composition is in the form of a single solution.

12. An enzyme composition of claim 11, wherein the concentration of cellulase for biopolishing is in the range of from 0.01 mg protein/l to 1g protein/l of the total solution.

13. An enzyme composition of claims 11 or 12, wherein the concentration of catalase is in the range of from 0.001 mg protein/l to 1 g protein/l of the total solution.
